# EUROPEAN PATENT SPECIFICATION

(11) **EP 3 209 718 B1**
(45) Date of publication and mention of the grant of the patent: **07.06.2023**
(21) Application number: 15853222.6
(22) Date of filing: 15.10.2015
(51) Int. Cl.: C08L 5/00

(54) **TISSUE-MIMICKING HYDROGEL COMPOSITIONS FOR BIOFABRICATION**
GEWEBEIMITIERENDE HYDROGELZUSAMMENSETZUNGEN FÜR BIOFABRIKATION
COMPOSITIONS D'HYDROGEL IMITANT UN TISSU POUR BIOFABRICATION

(30) Priority: 24.10.2014 US 201462068218 P
(43) Date of publication of application: 30.08.2017
(73) Proprietor: Wake Forest University Health Sciences, Winston-Salem, NC 27157 (US)
(72) Inventor: SKARDAL, Aleksander, Winston-Salem, North Carolina 27103 (US); SOKER, Shay, Greensboro, North Carolina 27410 (US)
(74) Representative: Marks & Clerk LLP
(86) International application number: PCT/US2015/055699
(87) International publication number: WO 2016/064648

(56) References cited:
- WO-A1-2011/088213
- WO-A1-2013/040559
- WO-A2-2014/040026
- US-A1- 2009 117 087
- US-A1- 2013 172 985
- US-A1- 2013 172 985
- US-A1- 2014 012 225
- US-A1- 2014 341 842
- IBRAHIM M. EL-SHERBINY ET AL: "Hydrogel scaffolds for tissue engineering: Progress and challenges", GLOBAL CARDIOLOGY SCIENCE & PRACTICE 2013, vol. 2013, no. 3, 1 September 2013 (2013-09-01), page 38, XP055441085, ISSN: 2305-7823, DOI: 10.5339/gcsp.2013.38
- TSANG ET AL.: 'THREE DIMENSIONAL TISSUE FABRICATION' ADV. DRUG DEL. REV vol. 56, no. 11, 22 September 2004, pages 1635 - 1647, XP004550361 DOI: 10.1016/J.ADDR.2004.05.001
- O'BRIEN ET AL: 'THREE-DIMENTIONAL PRINTING OF NANOMATERIAL SCAFFOLDS FOR COMPLEX TISSUE REGENRATION' TISSUE ENGINEERING PART B: REVIEWS, [Online] vol. 21, no. 1, 01 January 2015, pages 103 - 114, XP055435780
- Jiyuan Yang ET AL: "Synthesis and Characterization of Enzymatically Degradable PEG-Based Peptide-Containing Hydrogels", Macromolecular Bioscience, vol. 10, no. 4, 8 April 2010 (2010-04-08), pages 445-454, XP055526351, DE ISSN: 1616-5187, DOI: 10.1002/mabi.200900295

## Description

### Field of the Invention

The present invention concerns hydrogel "bioink" compositions useful for fabrication of artificial tissue constructs, methods of using the same, and products formed therefrom.

### Background of the Invention

Biofabrication technologies have emerged as tissue engineering approaches for building organs and organoids or tissue constructs. The combination of biocompatible materials and rapid prototyping provides a way to address the intricacies needed in viable tissues. One of the hurdles associated with biofabrication is the interfacing between the deposition/fabrication hardware and different types of biomaterials (or "bio-inks") being deposited. Standard hydrogels pose design problems because they are either printed as fluid solutions, limiting mechanical properties, or printed as solid hydrogels and broken up upon the extrusion process.

Hydrogel compositions for use in tissue engineering applications are described in WO2014/040026 (Murphy et al), WO 2011/088213 A1 (Skardal et al), US 2013/172985 A1 (Prestwich et al) and Ibrahim et al ("Hydrogel scaffolds for tissue engineering: Progress and challenges", Global Cardiology Science and Practice 2013, no. 3, 1 September 2013, page 38).

### Summary of the Invention

Embodiments of the materials described herein address the issues noted above by being extrudable, and by possessing a post-deposition or secondary crosslinking step which stabilizes and increases the stiffness of the end product to match a range of tissue types. Additionally, these "bioink" compositions can be supplemented with biochemical factors derived from tissues that result in a biochemical environment more like that of an *in vivo* tissue that cells in the biofabricated constructs then experience. These factors - both biochemical and mechanical - can increase the ability to maintain viable cells in culture and to increase their functionality for the duration of culture.

In view of the foregoing, the present invention provides an extrudable hydrogel composition (or "bioink") useful for making a three-dimensional organ construct according to claim 1. The composition comprises:
(a) a cross-linkable prepolymer; and
(b) a post-deposition crosslinking group (also referred to as a second crosslinking group) as defined in claim 1.

The composition may comprise: (c) optionally, but in some embodiments preferably, an initiator that catalyzes the reaction between said prepolymer and said post-deposition crosslinking group;
(d) live cells (*e.g.,* live animal cells); and
(e) optionally, but in some embodiments preferably, at least one growth factor.

The composition further comprises:
(f) water to balance.

According to further aspects of the invention there is provided a method of making a three-dimensional organ construct according to claims 8 to 11 and a device useful for modeling cellular function *in vitro* according to claims 12 to 15.

Some embodiments of the invention provide advantages as follows:
*Control over biochemical properties.* Tissues in the body have complex compositions: Various subpopulations of cells secrete signaling molecules such as growth factors and other cytokines that aid in maintaining viability and function of cells in tissues. Extracellular matrix is comprised of proteins and polymers that provide structure to the tissue and also interact with cell receptors acting as another type of signaling. Additionally, some ECM components bind growth factors (heparin, heparan sulfate) and slowly release them to the cells over time. The combination of these signals varies from tissue to tissue. We previously developed a method for providing components specific to the liver within a hydrogel in order to support primary human hepatocytes. By decellularizing any tissue, pulverizing it, dissolving it, we can produce tissue-specific biochemical signals from any tissue to cells in 3-D hydrogel constructs.
*Control over mechanical properties.* Mechanical properties, specifically elastic modulus, are important for 2 major reasons. First, as has been described in earlier reports, control over the hydrogel bioink stiffness allows for extrusion-based biofabrication using a soft gel, which can then be stiffened afterwards by secondary crosslinking to increase stability. Second, this second crosslinking step can be used to reach elastic modulus levels that are consistent with the target organ type for each individual organoid. For example, we can customize the liver bioinks to reach stiffnesses of 5-10 kPa, like a native liver, or cardiac bioinks (or microenvironment) to reach stiffnesses of 10-15 kPa like native cardiac tissue, in theory increasing the ability of these organoids to function in a similar manner to their native tissue counterparts.

The present invention is explained in greater detail in the drawings herein and the specification set forth below.

### Brief Description of the Drawings

**Figure 1****.** Analysis of components present in tissue ECM-derived solutions for providing biochemical factors to hydrogel bioinks. A) A panel displaying the growth factors and cytokines amounts (pg/ml) measured in liver, cardiac, and skeletal muscle ECM solutions. B) The concentrations of collagen, GAGs, and elastin in liver, cardiac, and skeletal muscle ECM solutions.
**Figure 2****.** A) Strategy of formulation of printable bioinks comprised of acrylate-based crosslinkers (crosslinker 1), alkyne-based crosslinkers (crosslinker 2), thiolated HA, thiolated gelatin, and unmodified HA and gelatin. B) Implementation of bioprintable hydrogel bioinks. The bioink formulation is prepared and spontaneously crosslinks through thiol-acrylate binding, resulting in a soft, extrudable material. Bioprinting is performed. Lastly, the bioprinted structures are fused, stabilized, and brought to the target stiffness.
**Figure 3****.** Bioprinting testing of bioinks. A) A 7 x 7 mm pattern used for bioink deposition testing in the bioprinter. B) An initial formulation of a PEGDA and 4-arm PEG alkyne containing bioink after printing. C) Improved extrusion and end structure smoothness after addition of unmodified HA and gelatin to improve shear thinning and material smoothing.
**Figure 4****.** Bioink stiffness control and range of formulations. A) Demonstration of the capability to control bioink stiffness using Gel # 2 in this panel. After stage 1 crosslinking, the gel is relatively soft and able to be extruded smoothly. After stage 2 crosslinking by UV light, stiffness increases by more than an order of magnitude. B) A range of final stiffness levels of a variety of bioink formulations after stage 2 crosslinking, spanning from approximately 100 Pa to approximately 20 kPa.
**Figure 5****.** Design of biofabricated organoids.
**Figure 6****.** LIVE/DEAD imaging of organoids after extrusion biofabrication in compositions of the invention.
**Figure 7****.** Albumin and Urea analysis of organoids after extrusion biofabrication in compositions of the invention.
**Figure 8****.** Viability of acetaminophen (paracetamol; N-acetyl-p-aminophenol; "APAP") treated organoids assessed by LIVE/DEAD staining.
**Figure 9****.** Albumin and Urea analysis of organoids after APAP treatment.

### Detailed Description of Illustrative Embodiments

The present invention is now described more fully hereinafter with reference to the accompanying drawings, in which embodiments of the invention are shown. This invention may, however, be embodied in many different forms within the scope of the appended claims and should not be construed as limited to the embodiments set forth herein; rather these embodiments are provided so that this disclosure will be thorough and complete and will fully convey the scope of the invention to those skilled in the art.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

### A. COMPOSITIONS.

Compositions of the present invention may comprise live cells in a "bioink," where the "bioink" is in turn comprised of a cross-linkable polymer, a post-deposition crosslinking group or agent, water; and other optional ingredients, including but not limited to growth factors, initiators (*e.g*., of cross-linking) etc. The compositions are in the form of a hydrogel. Various components and properties of the compositions are discussed further below.

***Cells**.* Any type of cells, generally live cells, may be used to carry out the present invention, including but not limited to plant, animal, and microbial cells (*e.g*., yeast, bacteria, etc.). The cells may be combinations of multiple cell types, including combinations of cells from the same organism or species, symbiotic combinations of cells of different species, etc. In general the cells are preferably animal cells (*e.g*., bird, reptile, amphibian, etc.) and in some embodiments are preferably mammalian cells (*e.g*., dog, cat, mouse, rat, monkey, ape, human). The cells may be differentiated or undifferentiated cells, but are in some embodiments tissue cells (*e.g*., liver cells such as hepatocytes, pancreatic cells, cardiac muscle cells, skeletal muscle cells, etc.). Where tissue cells are employed, they may be incorporated as one cell type of multiple cell types for that tissue, and may be incorporated as discrete cells, or as cell aggregates such as organoids (which organoids may be unencapsulated or encapsulated; *e.g.,* spheroids).

The cells may be incorporated into the composition in any suitable form, including as unencapsulated cells, or as cells previously encapsulated in spheroids. Animal tissue cells encapsulated or contained in polymer spheroids can be produced in accordance with known techniques, or in some cases are commercially available (*see, e.g.,* Insphero AG, 3D Hepg2 Liver Microtissue Spheroids (2012); Inspherio AG, 3D InSight™ Human Liver Microtissues, (2012)).

***Cross-linkable prepolymers.*** The prepolymer used to carry out the present invention can be further cross-linked to increase the elastic modulus thereof after deposition when employed in the methods described herein.

The prepolymer is formed from the at least partial crosslinking reaction of *(i)* a thiol-substituted oligosaccharide, a thiol-substituted hyaluronic acid, a thiol-substituted collagen, or a thiol-substituted gelatin and *(ii)* a first a thiol-reactive crosslinking agent, such as polyalkylene glycol diacrylate, polyalkylene glycol methacrylate, etc., and particularly polyethylene glycol diacrylate, etc.; thiolated crosslinking agent to create thiol-thiol disulfide bonds; gold nanoparticles gold functionalized crosslinkers forming thiol-gold bonds; etc., including combinations thereof.

***Cross-linking group.*** The compositions include a post-deposition crosslinking group which is a second thiol-reactive crosslinking agent comprising an alkyne. Suitable crosslinking groups include but are not limited to multi-arm thiol-reactive crosslinking agent, such as polyethylene glycol dialkyne, other alkyne-functionalized groups; etc., including combinations thereof.

***Initiators.*** Compositions of the invention may optionally, but in some embodiments preferably, include an initiator (*e.g.,* a thermal or photoinitiator). Any suitable initiator that catalyzes the reaction between said prepolymer and the second (or post-deposition) crosslinking group (*e.g.,* upon heating or upon exposure to light), may be employed.

***Growth factors.*** Compositions of the invention may optionally, but in some embodiments preferably, include at least one growth factor (*e.g.,* appropriate for the particular cells included, and/or for the particular tissue substitute being produced). An example is a decellularized extracellular matrix composition ("ECM") from a tissue corresponding to the tissue cells (*e.g.,* decellularized extracellular liver matrix when the live animal cells are liver cells; decellularized extracellular cardiac muscle matrix when the live animal cells are cardiac muscle cells; decellularized skeletal muscle matrix when the live animal cells are skeletal muscle cells; etc.). Additional collagens, glycosaminoglycans, and/or elastin (*e.g.,* which may be added to supplement the extracellular matrix composition), etc., may also be included.

***Elastic modulus.*** The composition preferably has an elastic modulus, at room temperature and atmospheric pressure, sufficiently low such that it can be manipulated and deposited on a substrate by whatever deposition method is employed (*e.g.,* extrusion deposition). Further, the composition optionally, but in some embodiments preferably, has an elastic modulus, again at room temperature and atmospheric pressure, sufficiently high so that it will substantially retain the shape or configuration in which it is deposited until subsequent cross-linking (whether that cross-linking be spontaneous, thermal or photoinitiated, etc.). In some embodiments, the composition, prior to deposition, has a stiffness of from 0.05, 0.1 or 0.5 to 1, 5 or 10 kiloPascals, or more, at room temperature and atmospheric pressure.

### B. METHODS.

The compositions can be used in a method of making a three-dimensional organ construct as defined in claim 8. The method comprises the steps of:
(a) providing a reservoir containing an extrudable hydrogel composition as described above, said composition further comprising live cells; then
*(b)* depositing the hydrogel composition onto a substrate (*e.g.*, by extrusion through a syringe); and then
*(c)* cross-linking said prepolymer with said second thiol-reactive crosslinking group comprising an alkyne by an amount sufficient to increase the stiffness of said hydrogel and form said three-dimensional organ construct *(e.g.,* by heating the hydrogel, irradiating the hydrogel composition with light (*e.g*., ambient light, UV light), altering the pH of the hydrogel; etc.).

The depositing step may be carried out with any suitable apparatus, including but not limited to that described in H.-W. Kang, S. J. Lee, A. Atala and J. J. Yoo, US Patent Application Pub. No. US 2012/0089238 (April 12, 2012). In some embodiments, the depositing step is a patterned depositing step: That is, deposition is carried out so that the deposited composition is deposited in the form of a regular or irregular pattern, such as a regular or irregular lattice, grid, spiral, etc.

In some embodiments, the cross-linking step increases the stiffness of said hydrogel by from 1 or 5 to 10, 20 or 50 kiloPascals, or more, at room temperature and atmospheric pressure.

In some embodiments, the hydrogel has a stiffness after said cross-linking step *(c)* of from 1 or 5 to 10, 20 or 50 kiloPascals at room temperature and atmospheric pressure.

In some embodiments, the method further comprises the step of depositing a supporting polymer (*e.g.,* poly-L-lactic acid, poly(glycolic acid), polycaprolactone; polystyrene; polyethylene glycol, etc., including copolymers thereof such as poly(lactic-co-glycolic acid),) on said substrate in a position adjacent that of said hydrogel composition (*e.g.,* concurrently with, after, or in alternating repetitions with, the step of depositing said hydrogel, and in some embodiments prior to the cross-linking step).

Any suitable substrate can be used for the deposition, including organic and inorganic substrates, and including substrates with or without features such as well, chambers, or channels formed thereon. For the particular products described below, the substrate may comprise a microfluidic device having at least one chamber (the chamber optionally but preferably associated with an inlet channel and/or an outlet channel), and the depositing is carried out in at least one chamber. In an alternative, the substrate may comprise a first planar member (*e.g.,* a microscope cover slip), the depositing step may be carried out that planar member, and the method may further comprise the step of inserting that planar member into a chamber of a microfluidic device. Post-processing steps, such as sealing of chambers, and maintaining the viability of cells, may be carried out in accordance with known techniques.

### C. PRODUCTS.

The methods and compositions described above can be used to make a device useful for modeling animal tissue function (such as liver function) *in vitro* as defined in claim 12. The device comprises: (a) a device body which is a microfluidic device having at least one chamber formed therein; (b) a hydrogel composition as described herein deposited in said chamber in a first pattern, (c) live animal tissue cells in said hydrogel composition; and (d) a structural support polymer deposited in said chamber adjacent said hydrogel. As noted above, the cells for such a device may comprise animal tissue cells, such as liver cells (*e.g.*, hepatocytes), pancreatic cells, skeletal muscle cells; cardiac muscle cells, etc.).

The device body or microfluidic device may itself be formed of any suitable material or combination of materials. Examples include, but are not limited to, polydimethylsiloxane (PDMS), polystyrene, polymethyl methacrylate (PMMA), polyacrylamide, polyethylene glycol (PEG) including functionalized PEG (e.g. PEG diacrylate, PEG diacrylamide, PEG dimethacrylate, etc., or any of the foregoing PEGs in in multi-arm forms, etc.), natural polymers or proteins that can be cross-linked or cured (*e.g.*, hyaluronic acid, gelatin, chondroitin sulfate, alginate, etc., including derivatives thereof that are functionalized with chemical groups to support cross linking, and including any of the "cross-linkable prepolymers" described above in cross-linked form, and combinations thereof. The device body may be formed by any suitable process, including molding, casting, additive manufacturing (3d printing), lithography, etc., including combinations thereof.

Where a structural support is included in the device as noted in the "Methods" section above, that structural support, like the hydrogel, may be patterned (*e.g.,* a regular or irregular pattern, such as a regular or irregular lattice, grid, spiral, etc.).

In some embodiments, the tissue cells are contained in spheroids (*e.g.,* polymer spheroids), which spheroids are contained in said hydrogel.

As noted above, the hydrogel is preferably cross-linked following deposition, such that the hydrogel residing in the device has a stiffness of from 1 or 5 to 10, 20 or 50 kiloPascals at room temperature and atmospheric pressure (*e.g.,* preferably corresponding to the natural tissue in which the cells are found *in vivo*).

The device may be provided as a cartridge, or as a subcombination unit or "building block" configured in a manner suitable for "snap in" installation in a larger apparatus including pumps, detectors, or the like, as discussed further below.

### D. PACKAGING, STORAGE AND SHIPPING.

Once produced, subcombination or "cartridge" devices as described above may be used immediately, or prepared for storage and/or transport.

To store and transport the product, a transient protective support media that is a flowable liquid at room temperature (*e.g.,* 25° C), but gels or solidifies at refrigerated temperatures (*e.g.,* 4° C), such as a gelatin mixed with water, is added into the device to substantially or completely fill the chamber(s), and preferably also any associated conduits. Any inlet and outlet ports are capped with a suitable capping element (*e.g.,* a plug) or capping material (*e.g.,* wax). The device is then packaged together with a cooling element (*e.g*., ice, dry ice, a thermoelectric chiller, etc.) and all placed in a (preferably insulated) package.

Alternatively, to store and transport the product, a transient protective support media that is a flowable liquid at cooled temperature (*e.g.,* 4° C), but gels or solidifies at warmed temperatures such as room temperature (*e.g.,* 20° C) or body temperature (e.g., 37° C), such as poly(N-isopropylacrylamide and poly(ethylene glycol) block co-polymers.

Upon receipt, the end user simply removes the device from the associated package and cooling element, allows the temperature to rise or fall (depending on the choice of transient protective support media), uncaps any ports, and removes the transient protective support media with a syringe (*e.g.,* by flushing with growth media).

The present invention is explained in greater detail in the following non-limiting Examples.

### EXAMPLES

### Materials and Methods

*Materials.* Hydrochloric acid (HCl) was from Fischer Scientific (Houston, TX). Pepsin (porcine gastic mucosa) was from Sigma Aldrich (St. Louis, MO, USA). Heprasil (est. 160 kDa MW), Gelin-S, and Extralink (PEGDA, 3.4 kDa MW) were used from HyStem-HP hydrogel kits from ESI-BIO (Alameda, California, USA). PEG 4-Arm Acrylate (10 and 20 kDa MW), PEG 4-Arm Alkyne (10 kDa MW), and PEG 8-Arm Alkyne (10 kDa MW) were from Creative PEGWorks (Winston-Salem, North Carolina, USA).

*Preparation of tissue-specific extracellular matrix (ECM) digest.* Tissue-specific ECM digest solutions were prepared as previously described for liver (A. Skardal, L. Smith, S. Bharadwaj, A. Atala, S. Soker and Y. Zhang, Biomaterials, 33, 4565 (2012).). Fresh liver, cardiac, or skeletal muscle tissue was rinsed with chilled Dulbecco's phosphate buffered saline (DPBS). The tissues were cut into 10 cm by 0.5 - 1.0 cm strips and minced with surgical scalpels. Minced tissue was transferred to 500 ml distilled water and shook on a rotary shaker at 200 rpm for 3 days at 4°C, during which the water was changed three times per day. The tissues were treated with 2% Triton X-100 for 4 days followed by 2% TX-100 with 0.1% NH4OH for 24 h. During the TX-100 rinses, solutions were changed twice daily. The decellularized tissues were washed for 2 additional days in distilled water to remove any traces of TX-100, after which they were stored at 4°C until further use.

Decellularized tissue ECMs were frozen and lyophilized for 48 h. Following lyophilization, samples were ground into a powder with a freezer mill. One gram of liver tissue or liver ECM powder was mixed with 100 mg Pepsin (Porcine gastric mucosa, 3400 units of protein, Fisher Scientific, Fair Lawn, NJ) and sterilized by gamma irradiation (1 Mrad). All subsequent procedures following sterilization were carried out under sterile conditions. Hydrochloric acid (0.1 N, 100 mL) was added to the sterilized materials and incubated for 48 h at room temperature. The resulting mixture was transferred to a 50 ml conical tube and centrifuged at 3000 rpm for 15 min. The supernatant was removed and the pellet was discarded. This was repeated 3 times until the supernatant was clear. To ensure there was no more particulate matter remaining, the suspension was filtered through a 0.45 mm syringe filter (Fisher Scientific). The resulting decellularized ECM solutions were stored at 80°C until further use.

*Hydrogel bioink formulations and preparation.* Prior to hydrogel formulation, a photonitiator, Irgacure 2959 (4-(2-hydroxyethoxy)phenyl-(2-propyl)ketone, Sigma), was dissolved in water at 0.05% w/v. To form hydrogel bioinks, first the base material components from HyStem-HP hydrogel kits (ESI-BIO, Alameda, CA) were dissolved in the water-phoinitiator solution. Briefly, Heprasil and Gelin-S were dissolved in water-phoinitiator solution to make 2% w/v solutions. Extralink, the crosslinker, was dissolved in water-phoinitiator solution to make a 4% and 8% w/v solution. Additionally, multi-arm PEG-based crosslinkers were prepared separately: PEG 4-Arm Acrylate (10 kDa or 20 kDa MW; 4% and 8% w/v), PEG 4-Arm Alkyne (10 kDa MW; 4% w/v), and PEG 8-Arm Alkyne (10 kDa MW; 8%, 10%, 16% and 20% w/v).

Following dissolution of all materials, hydrogels were formulated by 2 general schemes. In the first, 4 parts 2% Heprasil, 4 parts 2% Gelin-S, 1 part crosslinker 1, 1 part crosslinker 2 is combined with 10 parts tissue ECM solution (or water as a generic non-tissue-specific hydrogel). The resulting mixture is vortexed to mix prior to use. For extrusion or bioprinting testing, the mixture is transferred into a syringe or printer cartridge and allowed to crosslink spontaneously for 30 minutes (stage 1 crosslinking). For rheological measurements, the mixture is transferred into a 35 mm petri dish and allowed to crosslink. In the second formulation approach, the Heprasil, Gelin-S, and crosslinker solution is not further diluted with tissue ECM solution or water in order to achieve an increased polymer concentration. Instead, the photoinitiator is dissolved in the tissue ECM solutions at 0.05% w/v, which subsequently is used to dissolve the Heprasil, Gelin-S, and various crosslinkers. These components are then combined in the same 4:4:1:1 volume ratio. The materials were transferred into syringes, printer cartridges or petri dishes and allowed to spontaneously crosslink (stage 1 crosslinking) as described above for implementation. For secondary crosslinking (stage 2) the stage 1-crosslinked gels are irradiated with ultraviolet light (365 nm, 18 w/cm²) to initiate a thiol-alkyne polymerization reaction.

*Printer compatibility testing.* Extrusion-based bioprinting was tested first on the laboratory bench with simple extrusion tests using standard syringes and small gauge needle tips (20 - 30 gauge). Next, bioink preparations were loaded into printer cartridges, allowed to undergo spontaneous stage 1 crosslinking, and extrusion compatibility for bioprinting was assessed using a custom 3-D bioprinting device designed in house specifically for tissue construct printing (*See, e.g.,* H. Kang, S. Lee, A. Atala and J.Yoo, US Patent Application Pub. No. US 2012/0089238 (April 12, 2012)). A 7 x 7 mm pattern was implemented for testing purposes. To improve shear thinning and extrusion properties, unmodified HA and gelatin was supplemented to the bioinks (1.5 mg/mL and 30 mg/mL) (Sigma). The tendency for the bioink to be extruded smoothly versus in irregular clumps was observed.

*Determination of bioink mechanical properties by rheology.* For determination of bioink mechanical properties, hydrogels were prepared as described above and pipetted into 35 mm petri dishes where they underwent the stage 1 spontaneous crosslinking. Rheological testing was performed using an HR-2 Discovery Rheometer (TA Instruments, Newcastle, DE). A 12-mm steel disc was lowered until contact with the surface of the hydrogel was made. The disc was lowered further until the axial force on the instrument, or normal force acting on the disc from the hydrogel, equaled 0.4 N. At this point G' was measured for each hydrogel using a shear stress sweep test ranging from 0.6 to 10 Pa at an oscillation frequency of 1 Hz applied by the rheometer.

For determination of the stiffness after completion of the second stage crosslinking, identical untested hydrogels were further crosslinked by UV photopolymerization after which G' measurement was performed as described.

*Bioprinting of liver organoids for biological validation of bioinks.* Primary liver hepatocyte-based spheroids were formed by hanging drop method. Spheroids were harvested and suspended within a liver-specific bioink formulation containing liver ECM materials, drawn into a syringe compatible with the bioprinting, and the bioink was allowed to spontaneously crosslink through thiol-acrylate bonding. After 30 minutes, the spheroid-containing bioink was bioprinted within a polycaprolactone support pattern on a plastic coverslip. Following bioink depostion, UV light was used to initiate the second crosslinking step, stabilizing the bioink further and raising the bioink stiffness to a value similar to native liver, thus comprising the larger liver organoid. As a control, a gelatin-based hydrogel previously used in the bioprinter was used to bioprint spheroids in parallel. Following printing, viability of the organoids was assessed using a LIVE/DEAD stain, after which the organoids were fixed in paraformaldehyde, and imaged with a Leica TCS LSI macro-confocal microscope to determine the relative amounts of viable (green fluorescent) and dead (red fluorescent) cells.

*Functional analysis of liver organoids and toxic insult.* Organoids were prepared as described above. 9 organoids were placed in microreactors for 14 day culture time courses, during which media aliquots would be sampled and reserved for functional analysis. Microreactors consist of polydimethylsiloxane (PDMS) devices with chambers for organoid placement, and channels through which cell culture media can be circulated from a reservoir using a micro-peristaltic pump. After sampling media aliquots on day 3 and day 6 for baseline functional metrics, 3 organoids continued in culture with normal media; 3 organoids were administered media containing 1 mM acetaminophen, and 3 organoids were administered 10 mM acetaminophen in media. Media aliquots were collected on days 10 and 14, after which urea and albumin secretion were quantified and viability was assessed by LIVE/DEAD imaging.

*Toxic insult and clinical relevant intervention with N-acetyl-L-cysteine.* Organoids were prepared again as described above and placed in microreactor devices for 14 days of culture. After sampling media aliquots on day 3 and day 6 for baseline functional metrics, 1 group of organoids continued in culture with normal media; another group of organoids was administered media containing 10 mM acetaminophen, and the third group of organoids was administered 10 mM acetaminophen plus 20 mM N-acetyl-L-cysteine (NAC) in media. Media aliquots were collected on day 10 and 14, after which urea and albumin secretion were quantified

### Results: Characterization.

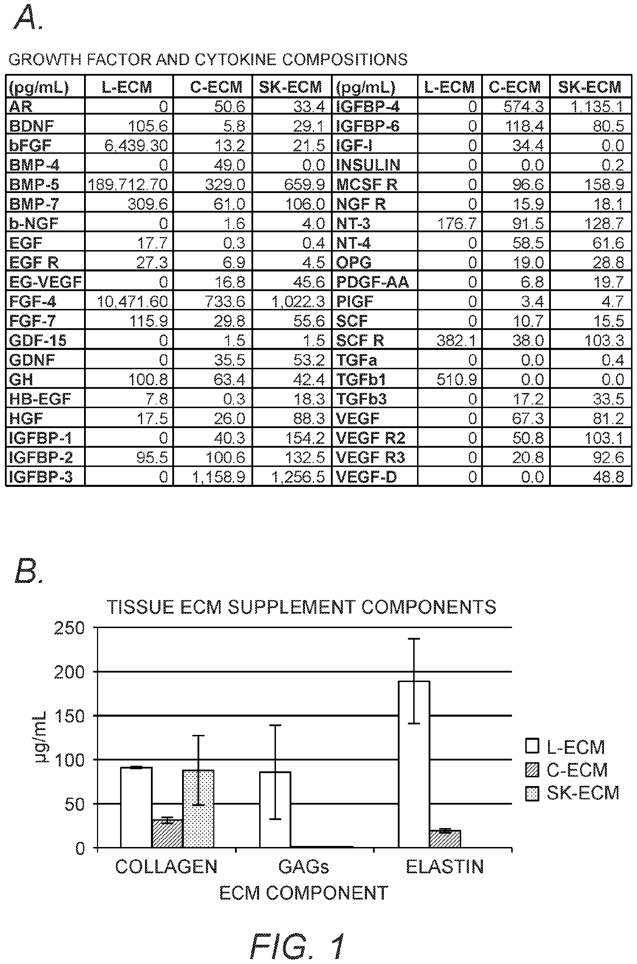

*ECM component analysis.* Liver ECM solutions were analyzed previously by a series of colorimetric assays (A. Skardal et al., *supra*). Two formulations were analyzed: 1) LEE, decellularized liver prepared as described above; and LTE, fresh liver tissue that was prepared identically, with the exception that it was not decellularized. The results revealed a clear trend, in which LEE solutions contained greater concentrations of collagen, glycosaminoglycans (GAGs), and elastin (**Figure 1A**). Specifically, the total collagen content of LEE, 91.33 mg/mL, was significantly greater than that of LTE, which was 4.17 mg/mL (p < 0.001), the elastin content of LEE, 189.33 mg/mL, was significantly greater than that of LTE, which was 36.00 mg/mL (p < 0.05) and the GAG content of LEE, 86.00 mg/ mL, was greater than that of LTE, which was 40.67 mg/mL, but not significantly (p > 0.05).

Cardiac and skeletal muscle ECM solutions (both decellularized preparations) were assessed in the same manner.

*Growth factor analysis.* Liver ECM solutions were analyzed previously by the Quantibody Growth Factor Array, which revealed that, in general, LEE contained higher concentrations of growth factors and cytokines (shown in pg/mL, **Figure 1B**). Of particular interest was that brain-derived neurotrophic factor (BDNF), bFGF, bone morphogenetic protein 5 (BMP-5), FGF-4, insulin-like growth factor binding protein 2 (IGFBP-2), and TGF-b1 were relatively conserved between both LEE and LTE. However, LEE also contained BMP-7, EGF, FGF-7, growth hormone (GH), heparin-binding EGF-like growth factor (HB-EGF), HGF and neurotrophin 3 (NT-3), which were not observed or were negligible in LTE. On the other hand, BMP-4, and glial-derived neurotrophic factor (GDNF) were present in LTE, but not in LEE (A Skardal et al., *supra*). Cardiac and skeletal muscle ECM solutions (both decellularized preparations) were assessed in the same manner.

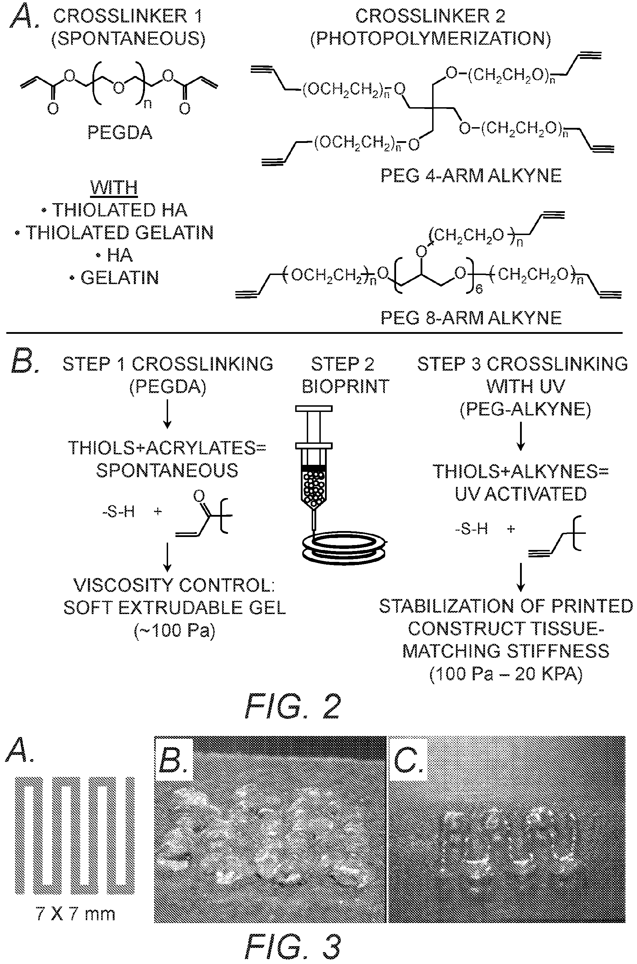

*Hydrogel bioink preparation and extrusion bioprinting testing.* Strategy and implementation of stage 1 and stage 2 crosslinking of the hydrogel bioinks is described in **Figure 2A and B**. A 7 x 7 mm pattern was implemented for testing purposes (**Figure 3A**). Initial tests showed that the initial formulations were extrudable, but appeared irregular and clumped during and after extrusion (**Figure 3B**). To improve shear thinning and extrusion properties, unmodified HA and gelatin was supplemented to the bioinks (1.5 mg/mL and 30 mg/mL). The improved smooth printed structure is shown in **Figure 3C**.

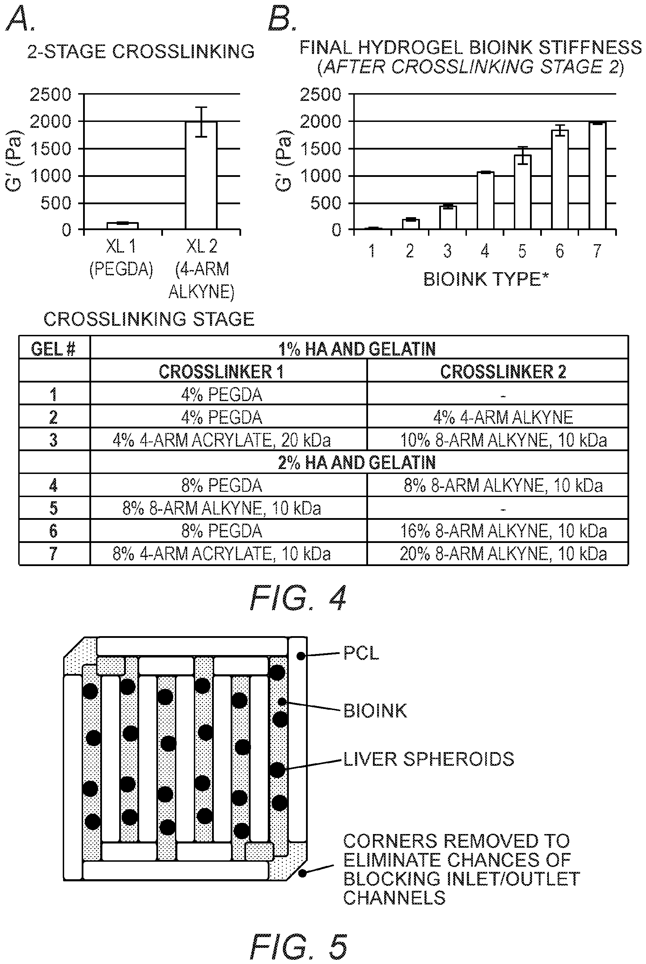

*Rheological testing.* As described in the methods, hydrogels of different formulations were prepared for rheological assessment of their mechanical properties. **Figure 4A** shows the increase in shear elastic modulus (***G*'**) in a gel that after spontaneous crosslinking with PEGDA had a G' of 113.66 Pa. This is the stage during which the hydrogel can be extruded as a bioink. After UV crosslinking with a 4-arm PEG Alkyne crosslinker, the ***G*'** value increases to 1981.79 Pa. **Figure 4B** shows the range of ***G*'** values that can be achieved through the secondary Alkyne-based crosslinking step, allowing mimicry of many tissue types in the body. **Table 1** shows a range of hydrogel stiffness within the range of this system, formulations, and associated tissue types.

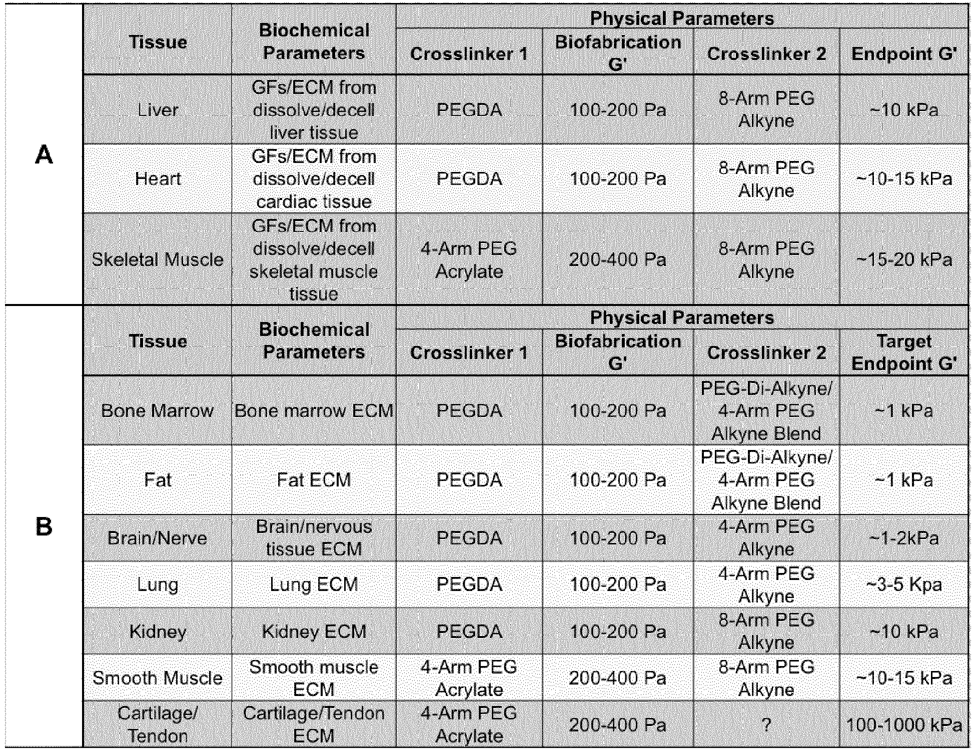

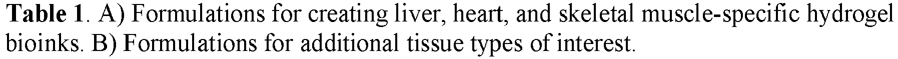

### Results: Validation.

*Bioink maintenance of primary organoid viability in a bioprinting biofabrication setting.* The biofabricated organoids were designed as depicted in **Figure 5**. Using the integrated printing approach of printing both polycaprolactone (PCL) and hydrogels, a PCL channel structure was printed inside of which the bioink with the liver cells was printed. The channel structures provide stability to the hydrogel when under flow as well as increasing the height-width aspect ratio of the hydrogel and cells. These structures were printed on plastic coverslips that were customized to fit inside the microfluidic microreactor chambers. These square coverslips feature 2 additional cuts in the corners to prevent occlusion of the microchannels providing the inlet and outlet flows to the organoid chamber in the microreactors.

Most spherical organoids within the overall construct stayed spherical during the printing process and maintained their original shape in culture. In earlier batches without PCL channels, some organoids were observed to become disfigured, compressed, or even torn during the printing process. This indicates a substantial improvement in the biofabrication technique. Uniformity of spherical organoid distribution and quantity was improved. In this batch, each organoid construct that was moved to microreactor culture (n = 9) contained between 40 - 45 spherical organoids. In past batches this number varied between 10 and 30.

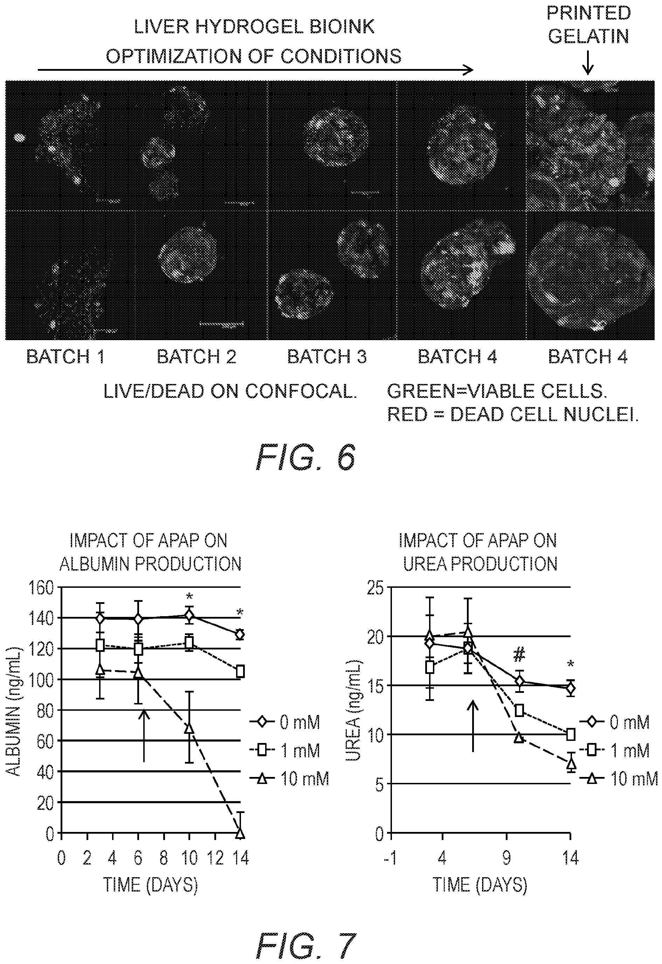

Multiple batches of organoids were biofabricated allowing opimization of the biofabrication conditions. Temperature was adjusted to remain near 37°C in the bioink and in the bioprinting chamber. Biofabrication preparation and methodology was performed in less time. Hepatocyte culture medium was added to the bioink to provide nutrients to the cells during printing. LIVE/DEAD imaging shows the increased viability of multiple iterations of the organoids after extrusion biofabrication in the bioink (**Figure 6**). A gelatin control was used in parallel with Batch 4 as a comparison. Simple gelatin gels are commonly used for extrusion biofabrication.

*Ability to support primary cell function in vitro using liver bioinks: Baseline secretory activity and toxic insult.* Liver organoids were prepared and biofabricated as described above, placed in microfluidic microreactors and cultured for 14 days. On day 6, organoids received normal media, or 1 of 2 concentrations of APAP.

Albumin analysis (**Figure 7**) by a Human Albumin ELISA Kit (Alpha Diagnostic International) revealed constant albumin production by bioprinted liver organoids through day 6, remaining on average near 120 ng/mL. It should be noted that during these 2 baseline timepoints we observed a trend in secretion magnitude, with 0 mM organoids secreting the most, followed by the 1 mM orgnanoid group, and then the 10 mM group. This decrease in baseline albumin production is believed to be due to the time at which the organoids were printed. The organoids in group 1 were printed first, and therefore have a slightly improved viability, which translates into improved albumin secretion. Despite this trend, albumin levels at these 2 time points were not statistically significant in comparison to one another. But in future studies, organoids will be randomly assigned to experimental treatment groups. Following APAP administration after day 6, albumin levels were significantly decreased in both the 1 mM and 10 mM groups compared to the 0 mM control (p < 0.05). Additionally, the 10 mM group albumin levels were significantly decreased compared to the 1 mM group (p < 0.01). In fact, at day 14 the albumin levels in the 10 mM group were nearly immeasurable.

Urea analysis (**Figure 7**) by a QuantiChrom Urea Colorimetric Assay Kit (BioAssay Systems) showed less drastic results than the albumin analysis, yet the results were still significant statistically with expected trends. Urea levels were not significantly different between the 3 groups during the time points prior to APAP administration. After APAP administration, measured urea levels appeared to drop in a dose dependent manner with respect to APAP concentration. On the day 10 time point, the 0 mM control group albumin level was significantly higher than both the 1 mM and 10 mM group (p < 0.05). On the day 14 time point, all 3 groups were significantly different from one another (p < 0.05)

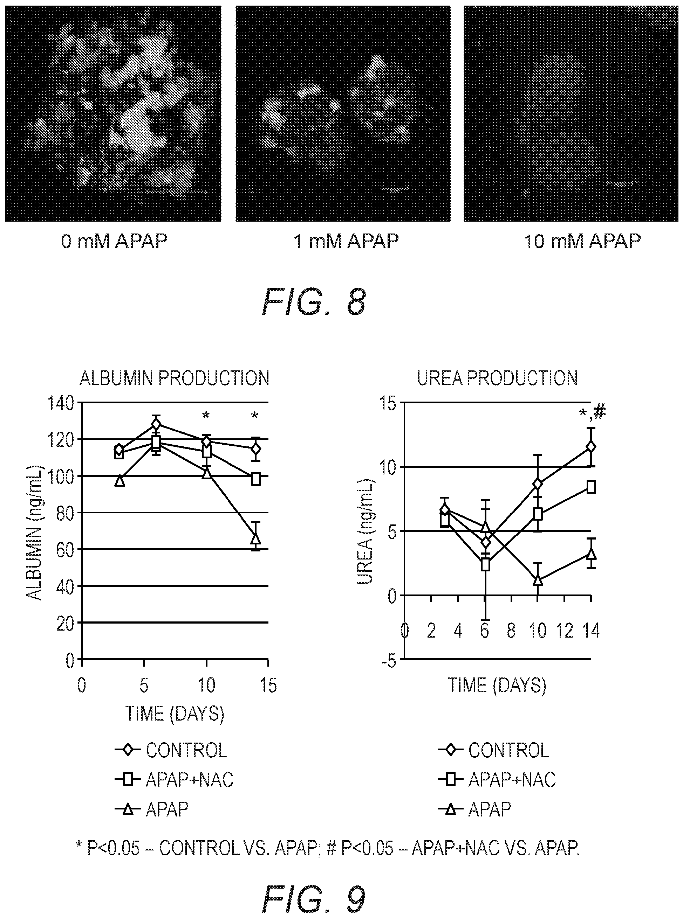

Viability of the 0 mM, 1 mM, and 10 mM APAP treated organoids was assessed by LIVE/DEAD staining and imaging using the macro-confocal microscope as has been described before (**Figure 8**). Based on the ratio of live cells to dead cells, it was evident that the 0 mM control group maintained a relatively high level of viability (70-90% at day 14) throughout the 14 day experiment. In comparison, the 1 mM group had decreased viability (30-50% at day 14), while the 0 mM group appeared to have nearly no viable cells at day 14.

*APAP toxicity testing and N-acetyl-L-cysteine intervention.* As described previously, liver organoids will prepared and bioprinted as described in previous reports. These organoids were used to set baseline functional metrics by media aliquots reserved on day 3 and day 6. Organoids would then undergo toxic insult by acetaminophen (10 mM), but some groups would also be administered N-acetyl-L-cysteine (20 mM) as a clinically relevant countermeasure. Media aliquots were reserved for functional analysis on days 3, 6, 10, and 14.

Albumin analysis (**Figure 9**) by a Human Albumin ELISA Kit (Alpha Diagnostic International) revealed constant albumin production by bioprinted liver organoids through day 6, remaining on average near 120 ng/mL, consistent with the experiment reported previously in the June report. Following administration of APAP only, we observed a decrease in detected albumin. This decrease was statistically significant (p < 0.5) compared to the untreated control organoids at day 10 and day 15. The co-administration of APAP and NAC saw a slight decrease in detected albumin production, decreasing to 98 ng/mL by day 14, however, this value was not significantly different that the control organoids nor the APAP only organoids. The general trend of the data was appropriate, suggesting that the liver organoids respond to APAP correctly, and can be rescued by NAC, as patients in the clinic might be.

Urea analysis (**Figure 9**) by a QuantiChrom Urea Colorimetric Assay Kit (BioAssay Systems) also showed results with promising trends. Following APAP administration, detected urea decreased as expected. In the control organoids, as well as the APAP + NAC organoids, detected urea production increased over time. There was no statistical significance between groups on day 3 and day 6 (to be expected), nor on day 10, despite the drop in APAP urea production. However, on day 14, both the control organoids and APAP + NAC organoids had increased detected urea levels (p < 0.05). As with the albumin data, these trends are appropriate and expected.

## Claims

1. An extrudable hydrogel composition comprising:
a cross-linkable prepolymer, the cross-linkable prepolymer being formed from a reaction of (i) a thiol-substituted oligosaccharide, a thiol-substituted hyaluronic acid, a thiol-substituted collagen or a thiol-substituted gelatin and (ii) a first thiol-reactive crosslinking agent;
a second thiol-reactive crosslinking agent comprising an alkyne; and
water.

2. The composition of claim 1, further comprising a thermal initiator or photoinitiator.

3. The composition of claim 1 or 2, wherein said second thiol-reactive crosslinking agent comprises a multi-arm thiol-reactive crosslinking agent.

4. The composition of any one of claims 1 to 3, wherein said cross-linkable prepolymer is formed from a reaction of a thiol-substituted hyaluronic acid and the first thiol-reactive crosslinking agent.

5. The composition of any one of claims 1 to 3, wherein said cross-linkable prepolymer is formed from a reaction of a thiol-substituted gelatin and the first thiol-reactive crosslinking agent.

6. The composition of any one of claims 1 to 5, wherein said composition has a stiffness of from 0.05, 0.1 or 0.5 to 1, 5 or 10 kilo Pascals, or more, at room temperature and atmospheric pressure.

7. The composition of any one of claims 1 to 6, further comprising live cells, optionally wherein said live cells are encapsulated in spheroids.

8. A method of making a three-dimensional organ construct, comprising the steps of:
(a) providing a reservoir containing an extrudable hydrogel composition of any one of claims 1 to 7, said composition further comprising: live cells; then
*(b)* depositing said hydrogel composition onto a substrate; and then
*(c)* cross-linking said cross-linkable prepolymer with said second thiol-reactive crosslinking agent by an amount sufficient to increase the stiffness of said hydrogel composition and form said three-dimensional organ construct.

9. The method of claim 8, wherein said crosslinking step is a thermally initiated or photoinitiated crosslinking step.

10. The method of any one of claims 8 to 9, wherein said hydrogel composition is sufficiently stiff to retain a configuration of deposition on said substrate from said depositing step to said cross-linking step, and/or
wherein:
*(i)* said hydrogel composition has a stiffness prior to said depositing step of from 0.05, 0.1 or 0.5 to 1, 5 or 10 kiloPascals, or more, at room temperature and atmospheric pressure; and/or
*(ii)* said cross-linking step increases the stiffness of said hydrogel composition by from 1 or 5 to 10, 20 or 50 kiloPascals, or more, at room temperature and atmospheric pressure; and/or
*(ii)* said hydrogel composition has a stiffness after said cross-linking step (c) of from 1 or 5 to 10, 20 or 50 kiloPascals at room temperature and atmospheric pressure.

11. The method of any one of claims 8 to 10, further comprising the step of:
*(d)* depositing a supporting polymer on said substrate in a position adjacent that of said hydrogel composition, and/or
wherein:
said substrate comprises a microfluidic device having at least one chamber, and said depositing is carried out in said at least one chamber; or
said substrate comprises a first planar member, said depositing step is carried out on said planar member, and said method further comprises the step of inserting said planar member into a chamber of a microfluidic device.

12. A device useful for modeling cellular function *in vitro*, comprising:
(a) a microfluidic device substrate having at least one chamber formed therein;
(b) the hydrogel composition of any one of claims 1 to 7 deposited in said chamber in a first pattern,
(c) live cells in said hydrogel composition; and
(d) a structural support polymer deposited in said chamber adjacent said hydrogel composition,
optionally wherein said structural support is patterned.

13. The device of claim 12, wherein said live cells are contained in spheroids, which spheroids are contained in said hydrogel composition.

14. The device of claim 12 or 13, wherein said hydrogel composition has a stiffness of from 1 or 5 to 10, 20 or 50 kiloPascals at room temperature and atmospheric pressure.

15. The device of any one of claims 12 to 14, packaged in a container with a transient protective support media in said chamber in gelled form, and optionally together with a cooling element in said container.

## Patentansprüche

1. Extrudierbare Hydrogelzusammensetzung umfassend:
ein vernetzbares Vorpolymer, wobei das vernetzbare Vorpolymer aus einer Reaktion von (i) einem thiolsubstituierten Oligosaccharid, einer thiolsubstituierten Hyaluronsäure, einem thiolsubstituierten Kollagen oder einer thiolsubstituierten Gelatine und (ii) einem ersten thiolreaktiven Vernetzungsmittel entsteht;
ein zweites thiolreaktives Vernetzungsmittel, das ein Alkyn umfasst; und
Wasser.

2. Zusammensetzung nach Anspruch 1, ferner einen Wärmeinitiator oder Fotoinitiator umfassend.

3. Zusammensetzung nach Anspruch 1 oder 2, wobei das zweite thiolreaktive Vernetzungsmittel ein thiolreaktives Multiarm-Vernetzungsmittel umfasst.

4. Zusammensetzung nach einem der Ansprüche 1 bis 3, wobei das vernetzbare Vorpolymer aus einer Reaktion von einer thiolsubstituierten Hyaluronsäure und dem ersten thiolreaktiven Vernetzungsmittel entsteht.

5. Zusammensetzung nach einem der Ansprüche 1 bis 3, wobei das vernetzbare Vorpolymer aus einer Reaktion von einer thiolsubstituierten Gelatine und dem ersten thiolreaktiven Vernetzungsmittel entsteht.

6. Zusammensetzung nach einem der Ansprüche 1 bis 5, wobei die Zusammensetzung eine Steifigkeit von 0,05, 0,1 oder 0,5 bis 1, 5 oder 10 Kilopascal oder mehr bei Raumtemperatur und Luftdruck aufweist.

7. Zusammensetzung nach einem der Ansprüche 1 bis 6, ferner lebende Zellen umfassend, wobei die lebenden Zellen in Sphäroiden eingekapselt sind.

8. Verfahren zum Herstellen eines dreidimensionalen Organkonstrukts, umfassend die Schritte des:
*(a)* Bereitstellens eines Reservoirs, das eine extrudierbare Hydrogelzusammensetzung nach einem der Ansprüche 1 bis 7 enthält, wobei die Zusammensetzung ferner lebende Zellen umfasst; dann
*(b)* Absetzens der Hydrogelzusammensetzung auf ein Substrat; und dann
*(c)* Vernetzens des vernetzbaren Vorpolymers mit dem zweiten thiolreaktiven Vernetzungsmittel in einer Menge, die zum Erhöhen der Steifigkeit der Hydrogelzusammensetzung und Bilden des dreidimensionalen Organkonstrukts ausreicht.

9. Verfahren nach Anspruch 8, wobei der Vernetzungsschritt ein wärmeinitiierter oder fotoinitiierter Vernetzungsschritt ist.

10. Verfahren nach einem der Ansprüche 8 bis 9, wobei die Hydrogelzusammensetzung ausreichend steif ist, eine Absetzkonfiguration auf dem Substrat von dem Absetzschritt bis zu dem Vernetzungsschritt beizubehalten, und/oder
wobei
*(i)* die Hydrogelzusammensetzung eine Steifigkeit vor dem Absetzschritt von 0,05, 0,1 oder 0,5 bis 1,5 oder 10 Kilopascal oder mehr bei Raumtemperatur und Luftdruck aufweist; und/oder
*(ii)* der Vernetzungsschritt die Steifigkeit der Hydrogelzusammensetzung um 1 oder 5 bis 10, 20 oder 50 Kilopascal oder mehr bei Raumtemperatur und Luftdruck erhöht; und/oder
*(ii)* die Hydrogelzusammensetzung eine Steifigkeit nach dem Vernetzungsschritt *(c)* von 1 oder 5 bis 10, 20 oder 50 Kilopascal bei Raumtemperatur und Luftdruck aufweist.

11. Verfahren nach einem der Ansprüche 8 bis 10, ferner den Schritt umfassend des:
*(d)* Absetzens eines stützenden Polymers auf dem Substrat in einer Position neben derjenigen der Hydrogelzusammensetzung und/oder
wobei:
das Substrat eine mikrofluidische Vorrichtung umfasst, die mindestens eine Kammer aufweist, und das Absetzen in der mindestens einen Kammer ausgeführt wird; oder
das Substrat ein erstes planares Element umfasst, der Absetzschritt auf dem planaren Element ausgeführt wird und das Verfahren ferner den Schritt des Einführens des planaren Elements in eine Kammer einer mikrofluidischen Vorrichtung umfasst.

12. Vorrichtung, die zum Modellieren einer zellulären Funktion in vitro nützlich ist, umfassend:
(a) ein mikrofluidisches Vorrichtungssubstrat, das mindestens eine darin gebildete Kammer aufweist;
(b) die Hydrogelzusammensetzung nach einem der Ansprüche 1 bis 7, die in der Kammer in einem ersten Muster abgesetzt ist;
(c) lebende Zellen in der Hydrogelzusammensetzung; und
(d) ein strukturelles Stützpolymer, das in der Kammer neben der Hydrogelzusammensetzung abgesetzt wird,
wobei wahlweise die strukturelle Stütze gemustert ist.

13. Vorrichtung nach Anspruch 12, wobei die lebenden Zellen in Spheroiden enthalten sind, welche Sphäroide in der Hydrogelzusammensetzung enthalten sind.

14. Vorrichtung nach Anspruch 12 oder 13, wobei die Hydrogelzusammensetzung eine Steifigkeit von 1 oder 5 bis 10, 20 oder 50 Kilopascal bei Raumtemperatur und Luftdruck aufweist.

15. Vorrichtung nach einem der Ansprüche 12 bis 14, die in einem Behälter mit einem vergänglichen, schützenden Stützmedium in der Kammer in gelierter Form und wahlweise zusammen mit einem Kühlelement in dem Behälter verpackt ist.

## Revendications

1. Composition extrudable d'hydrogel comprenant :
un prépolymère réticulable, le prépolymère réticulable étant formé à partir d'une réaction (i) d'un oligosaccharide substitué par un groupe thiol, d'un acide hyaluronique substitué par un groupe thiol, d'un collagène substitué par un groupe thiol ou d'une gélatine substituée par un groupe thiol et (ii) d'un premier agent de réticulation réactif avec un groupe thiol ;
un deuxième agent de réticulation réactif avec un groupe thiol comprenant un alcyne ; et
de l'eau.

2. Composition selon la revendication 1, comprenant en outre un initiateur thermique ou un photoinitiateur.

3. Composition selon la revendication 1 ou 2, dans laquelle ledit deuxième agent de réticulation réactif avec un groupe thiol comprend un agent de réticulation à bras multiples réactif avec un groupe thiol.

4. Composition selon l'une quelconque des revendications 1 à 3, dans laquelle ledit prépolymère réticulable est formé à partir d'une réaction d'un acide hyaluronique substitué par un groupe thiol et du premier agent de réticulation réactif avec un groupe thiol.

5. Composition selon l'une quelconque des revendications 1 à 3, dans laquelle ledit prépolymère réticulable est formé à partir d'une réaction d'une gélatine substituée par un groupe thiol et du premier agent de réticulation réactif avec un groupe thiol.

6. Composition selon l'une quelconque des revendications 1 à 5, dans laquelle ladite composition a une rigidité de 0,05, 0,1 ou 0,5 à 1,5 ou de 10 kiloPascals, ou plus, à la température ambiante et à la pression atmosphérique.

7. Composition selon l'une quelconque des revendications 1 à 6, comprenant en outre des cellules vivantes, facultativement dans laquelle lesdites cellules vivantes sont encapsulées dans des sphéroïdes.

8. Procédé de fabrication d'une construction d'organe tridimensionnelle, comprenant les étapes de :
*(a)* fourniture d'un réservoir contenant une composition extrudable d'hydrogel selon l'une quelconque des revendications 1 à 7, ladite composition comprenant en outre : des cellules vivantes ; puis
*(b)* le dépôt de ladite composition d'hydrogel sur un substrat ; et ensuite
*(c)* la réticulation dudit prépolymère réticulable avec ledit deuxième agent de réticulation réactif avec un groupe thiol en une quantité suffisante pour accroître la rigidité de ladite composition d'hydrogel et former ladite construction d'organe tridimensionnelle.

9. Procédé selon la revendication 8, dans lequel ladite étape de réticulation est une étape de réticulation thermiquement initiée ou photoinitiée.

10. Procédé selon l'une quelconque des revendications 8 à 9, dans lequel ladite composition d'hydrogel est suffisamment rigide pour conserver une configuration de dépôt sur ledit substrat à partir de ladite étape de dépôt jusqu'à ladite étape de réticulation, et/ou
dans lequel :
*(i)* ladite composition d'hydrogel a une rigidité avant ladite étape de dépôt de 0,05, 0,1 ou 0,5 à 1,5 ou 10 kiloPascals, ou plus, à la température ambiante et à la pression atmosphérique ; et/ou
*(ii)* ladite étape de réticulation accroît la rigidité de ladite composition d'hydrogel de 1 ou 5 à 10, 20 ou 50 kiloPascals, ou plus, à la température ambiante et à la pression atmosphérique ; et/ou
*(ii)* ladite composition d'hydrogel a une rigidité après ladite étape de réticulation *(c)* de 1 ou 5 à 10, 20 ou 50 kiloPascals à la température ambiante et à la pression atmosphérique.

11. Procédé selon l'une quelconque des revendications 8 à 10, comprenant en outre l'étape de :
*(d)* dépôt d'un polymère de support sur ledit substrat dans une position adjacente à celle de ladite composition d'hydrogel, et/ou
dans lequel :
ledit substrat comprend un dispositif microfluidique ayant au moins une chambre, et ledit dépôt est effectué dans ladite au moins une chambre ; ou
ledit substrat comprend un premier élément plan, ladite étape de dépôt est effectuée sur ledit élément plan, et ledit procédé comprend en outre l'étape d'insertion dudit élément plan dans une chambre d'un dispositif microfluidique.

12. Dispositif utile pour la modélisation d'une fonction cellulaire *in vitro*, comprenant :
(a) un substrat dispositif microfluidique ayant au moins une chambre formée en son sein ;
(b) la composition d'hydrogel selon l'une quelconque des revendications 1 à 7 déposée dans ladite chambre en un premier motif,
(c) des cellules vivantes dans ladite composition d'hydrogel ; et
(d) un polymère de support structurel déposé dans ladite chambre adjacent à ladite composition d'hydrogel,
facultativement dans lequel ledit support structurel est structuré.

13. Dispositif selon la revendication 12, dans lequel lesdites cellules vivantes sont contenues dans des sphéroïdes, lesquels sphéroïdes sont contenus dans ladite composition d'hydrogel.

14. Dispositif selon la revendication 12 ou 13, dans lequel ladite composition d'hydrogel a une rigidité de 1 ou 5 à 10, 20 ou 50 kiloPascals à la température ambiante et à la pression atmosphérique.

15. Dispositif selon l'une quelconque des revendications 12 à 14, emballé dans un récipient avec un milieu de support protecteur temporaire dans ladite chambre sous une forme gélifiée, et facultativement conjointement à un élément refroidissant dans ledit récipient.
